# EUROPEAN PATENT SPECIFICATION

(11) **EP 0 578 849 B1**
(45) Date of publication and mention of the grant of the patent: **17.05.1995**
(21) Application number: 92112014.3
(22) Date of filing: 17.07.1992
(51) Int. Cl.: C07D 319/06, C07D 319/08, C07D 409/06

(54) **Process for the preparation of 1,3-dioxane-4,6-dione derivates**
Verfahren zur Herstellung von 1,3-Dioxan-4,6-Dionderivaten
Procédé pour la préparation de dérivés de 1,3-dioxane-4,6-dione

(43) Date of publication of application: 19.01.1994
(73) Proprietor: BIOGAL GYOGYSZERGYAR, 4042 Debrecen (HU)
(72) Inventor: Toth, György, Dr., H-4400 Nyiregyhaza (HU); Balint, Janos, Dr., H-4032 Debrecen (HU); Elek, Klara, H-4032 Debrecen (HU); Moricz, Zsuzsanna, H-4032 Debrecen (HU); Mudra, Eva, H-4211 Ebes (HU)
(74) Representative: Beszédes, Stephan G., Dr.

(56) References cited:
- TETRAHEDRON LETTERS, vol. 24, no. 45, 1983, Oxford, GB, pages 4951 - 4954 D.M. HRUBOWCHAK, ET AL.: 'The reductive alkylation of Meldrum's acid'
- SYNTHETIC COMMUNICATIONS, vol. 16, no. 13, 1986, New York, US, pages 1701 - 1707 X. HUANG, ET AL.: 'One pot synthesis of monosubstituted isopropylidene malonates'
- Houben-Weyl, vol. 4/1d, "Reduktion II", Stuttgart 1983, page 559
- Organic Reactions 15 (1967) pages 204-206

## Description

This invention relates to a new process for preparing 1,3-dioxane-4,6-dione derivatives of the general formula (I), 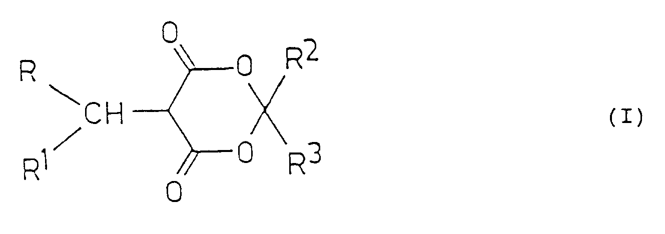
wherein
- R: means hydrogen, C₁₋₄alkyl or (C₁₋₅alkoxy)carbonyl group;
- R¹: stands for a C₁₋₆ alkyl, (C₁₋₅alkyl)carbonyl, (C₁₋₅alkoxy)carbonyl or (C₁₋₅alkoxy)carbonyl-(C₁₋₄-alkyl) group; or a cyclohexyl group; or a cyclopentyl group; or a phenyl, furyl or thienyl group, optionally mono-, di- or trisubstituted by halogen, C₁₋₄alkoxy, C₁₋₄alkyl, nitro, C₁₋₄alkylthio, di(C₁₋₄alkyl)amino or hydroxy groups; or a b-CH=CH group, wherein b is a phenyl group; or
- R and R¹: together form a straight-chain C₄₋₅alkylene group;
- R²: represents a C₁₋₅alkyl or phenyl group;
- R³: means hydrogen or a C₁₋₄alkyl group; or
- R² and R³: together form a pentylene group,
by reducing a compound of the general formula (IV), 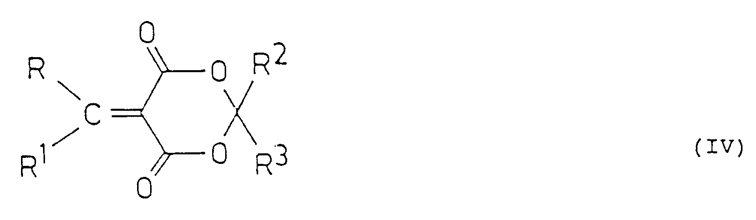
wherein R, R¹, R² and R³ are as defined above.

The compounds of general formula (IV) are preferably pepared by reacting an oxo compound of the general formula (II), 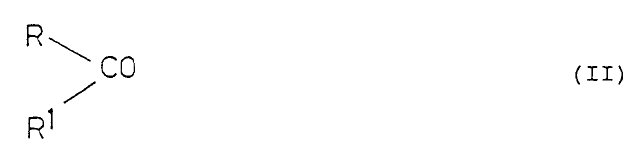
wherein R and R¹ are as defined above with an 1,3-dioxane-4,6-dione derivative of the general formula (III), 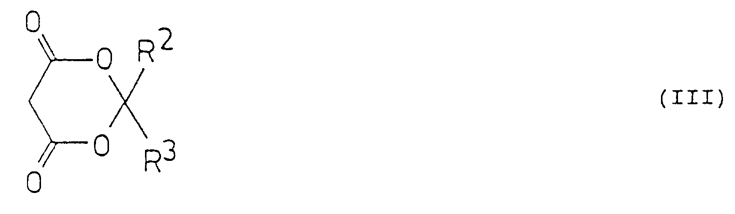
wherein R² and R³ are as defined above.

In the last years, the role of compounds of general formula (I) has become more important since they can be easily transformed to useful intermediates or final products in various ways [Heterocycles 32, 529 (1991)]: e.g. a group, being different from the substituent being present, can be introduced to the 5-position of the 1,3-dioxane-4,6-dione skeleton; furthermore, malonic acid and carboxylic acid derivatives can be obtained by cleaving the 1,3-dioxane-4,6-dione ring with suitable reagents [Chem Soc. Reviews 7, 345 (1978)].

The best known representative of compounds of the general formula (III) is the 2,2-dimethyl-1,3-dioxane-4,6-dione [(III), R² = R³ = CH₃; hereinafter named Meldrum's acid].

A number of publications have been devoted to the preparation of compounds of the general formula (I) by using Meldrum's acid as starting substance. However, when reacting Meldrum's acid with halide compounds in order to substitute it in 5-position, 5,5-disubstituted Meldrum's acid is obtained in each case, except a few, mostly very expensive halide compounds having a particular structure. The 5-mono-substituted Meldrum's acid derivatives according to the general formula (I) cannot be prepared in this way (Synthesis 1982, 452).

Thus, compounds of the general formula (I) are prepared by reacting a monosubstituted malonic acid with acetone and acetic anhydride in the presence of sulfuric acid as catalyst to give a 5-substituted 2,2-dimethyl-1,3-dioxane-4,6-dione derivative [(I), R¹ = R³= CH₃] [Chem. Ber. 94, 929 (1961); J. Am. Chem. Soc. 80, 4933 (1958)]. A disadvantage of this process is that the monosubstituted malonic acid used as starting substance can only be prepared by a multi-step synthesis in a low yield.

According to an other known method, the compounds of general formula (I) are prepared in such a way that an 1,3-dioxane-4,6-dione derivative of general formula (III) is reacted with an aldehyde or ketone of general formula (II); then the double bond of the unsaturated compound of general formula (IV) formed is saturated with hydrogen, e.g. by catalytic hydrogenation [J. Org. Chem. 26, 992 (1961)] or sodium borohydride (Tetrahedron Lett. 1979, 2325) or lithium aluminium hydride [Monatsh. Chem. 98, 184 (1967)] to give the compound of general formula (I).

The first step of the above process is a Knoevenagel-type condensation reaction rapidly proceeding with an aldehyde and significantly more slowly with a ketone. A disadvantage of this method is that, by using the so-called reactive aldehydes (e.g. benzaldehyde, straight-chain alkyl aldehydes) Michael-type bis-compounds are mainly formed in such a way that first a compound of the general formula (IV) is formed in a very rapid reaction, then a second molecule of the compound of general formula (III) is added to the double bond of the compound of general formula (IV) obtained (Tetrahedron Lett. 1969, 4983). The formation of the Michael adduct can be restricted by saturating in situ with hydrogen the double bond of the compound of general formula (IV) formed in the condensation reaction.

According to the literature, the compounds of general formula (I) are directly prepared from an aldehyde or ketone of the general formula (II) with a Meldrum's acid of the general formula (III) by reacting the components in the presence of piperidinium acetate or sodium hydrogen telluride [Synth. Commun. 16, 1701 (1986)] or without piperidinium acetate in the presence of borohydride/dimethylamine [Tetrahedron Lett. 24, 4951 (1983)].

The processes based on the reduction of compounds of the general formula (IV) are difficult to carry out since the compounds of general formulae (IV) and (I) are heat-sensitive and liable to decomposition. Another problem is that the reduction should be selectively accomplished in order to reduce only the double bond being present in 5-position of the compound of general formula (IV), without affecting any other double bond, e.g. the carbonyl or nitro group being present in the molecule.

The known processes involve a number of difficulties. The preparation from ketones of compounds of the general formula (IV) gives low yields in most cases. Catalytic hydrogenation requires a complicated and expensive equipment and metal catalysts are usually inflammable. Other particular reducing agents such as hydrides are commonly known to be inflammable and explosive; the reduction has to be carried out in an inert gas atmosphere.

These reactions require suitable safety measures and equipments. A further difficulty is that 1,3-dioxane-4,6-dione derivatives are commonly unstable to heat; thus, the reaction rate cannot be unrestrictedly increased by elevating the temperature; and the product obtained contains, as a consequence of decomposition, other contaminations, too.

Furthermore from HOUBEN-WEYL, Vol. 4/1d, "REDUKTION II", Stuttgart 1981, page 559 it has been known to reduce α, β-unsaturated ketones of formula 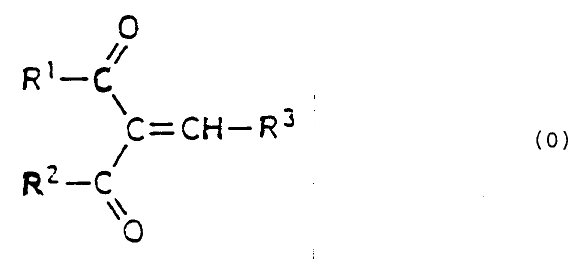
with triethylammonium formate at the high temperatures of 145 to 150°C to the corresponding saturated ketones.

Thus, the problem underlying to the present invention is to prepare the compounds of general formula (I) in a simple and selective manner at room temperature in good yields, by using the "one pot" method with safe reagents in a simple equipment.

The invention is based on the recognition that the compounds of general formula (IV) can easily and simply be reduced to the compounds of general formula (I) in the presence of a mixture containing formic acid and [a] secondary and/or tertiary amine(s).

Formic acid is a known reducing agent in the chemical literature, but compounds of the general formula (IV) cannot be reduced by formic acid alone. On using formic acid together with a palladium-on-carbon catalyst, all double bonds being present in the compound of general formula (IV) are reduced. Thus, it is surprising that, in the presence of a secondary or tertiary amine, formic acid is capable to selectively reduce compounds of the general formula (IV) to compounds of the general formula (I) already at room temperature in a good yield.

The invention is based on the further recognition that the condensation of compounds of general formula (II) with compounds of the general formula (III) is catalyzed by the mixture of formic acid and [a] secondary and/or tertiary amine(s); in the presence of these compounds the compounds of general formula (IV) can be obtained in a rapid reaction without formation of side-products, in a good yield, and the reduction step is not affected by the 1 molar equivalent of water liberated in the reaction. Consequently, it is made possible to prepare the compounds of general formula (I) without isolating the compounds of general formula (IV), directly by reacting a compound of the general formula (II) with a compound of the general formula (III) and in situ reducing the intermediate of general formula (IV) obtained.

Thus, the invention relates to a new process for the preparation of compounds of the general formula (I), wherein R, R¹, R² and R³ are as defined above, by reacting a compound of general formula (II) with a compound of general formula (III) and/or reducing a compound of the general formula (IV), wherein R, R¹, R² and R³ are as defined above, at a temperature of 10 to 70°C characterized by working in the presence of formic acid and [a] secondary and/or tertiary amine(s).

Surprisingly as opposed to the process described in HOUBEN-WEYL, Vol. 4/1d, "REDUKTION II", Stuttgart 1981, page 559 in the process according to the invention the reduction of the ketones of formula IV of more complicated structure can be achieved with good results at the considerably lower temperatures of 10 to 70°C, whereas in case the higher temperatures of the said reference would be applied the desired end products of formula I could not be obtained.

The compounds of general formula (IV) can suitably be prepared by reacting an oxo compound of the general formula (II), wherein R and R¹ are as defined above, with an 1,3-dioxane-4,6-dione derivative of the general formula (III), wherein R² and R³ are as defined above, in the presence of a mixture of formic acid and [a] secondary and or tertiary amine(s).

Under the compounds to be prepared by the process according to the invention compounds of general formula (I) are preferred, wherein
- R: means hydrogen, C₁₋₄alkyl or (C₁₋₄alkoxy)carbonyl group;
- R¹: stands for a C₁₋₅ alkyl, (C₁₋₄alkyl)carbonyl, (C₁₋₄alkoxy)carbonyl or (C₁₋₄alkoxy)carbonyl-(C₁₋₄alkyl) group; or a phenyl, furyl or thienyl group optionally mono-, di- or trisubstituted by fluorine, chlorine or bromine or a C₁₋₄alkyl, C₁₋₄alkoxy, nitro, hydroxy, C₁₋₄alkylthio or di(C₁₋₄alkyl)amino group; or
- R and R¹: together form a straight-chain C₄₋₅alkylene group;
- R²: represents a C₁₋₄alkyl or phenyl group;
- R³: means hydrogen or a C₁₋₄alkyl group; or
- R² and R³: together form a pentylene group.

In the present description "C₁₋₄alkyl group" means a straight or branched chain alkyl group, e.g. methyl, ethyl, propyl, isopropyl, butyl, isobutyl, tert-butyl or sec-butyl group.

The substituted phenyl group as R¹ is preferably characterized by the general formula (V) 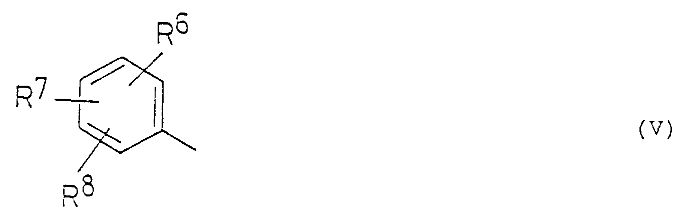
wherein R⁶, R⁷ and R⁸, being the same or different, stand for halogen, C₁₋₄alkyl, C₁₋₄alkoxy, hydroxy, nitro, C₁₋₄alkylthio or di(C₁₋₄alkyl)amino group.

The substituted furyl or thienyl group as R¹ is preferably characterized by the general formula (VI) 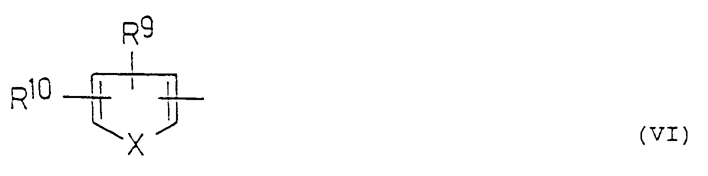
wherein
- X: stands for an oxygen or sulfur atom; and
- R⁹ and R¹⁰,: being the same or different, preferably mean hydrogen, chlorine, C₁₋₄alkyl, C₁₋₄alkoxy, nitro or hydroxy groups.

The compounds of general formula (IV) are not reduced by formic acid alone; for this purpose the presence of a secondary or tertiary amine is necessary. Useful secondary or tertiary amines are e.g. amines di- or tri-substituted by C₁₋₄alkyl group(s), such as dimethylamine, diethylamine, methylethylamine, trimethylamine, triethylamine or cyclic amines, e.g. pyridine, piperidine or N-(C₁₋₄alkyl)piperidines such as N-ethylpiperidine, as well as morpholine; from these bases triethylamine is the most useful.

For 1 mole of a compound of the general formula (IV) at least 1 mol of formic acid is required but it is suitable to use the formic acid in excess. It is preferable to use 1-20 moles, more preferably 3-12 moles and most preferably 6-12 moles of formic acid in relation to 1 mole of the compound of general formula (IV).

The molar ratio of the secondary or tertiary amine, respectively, to the formic acid may be varied between broad limits: the amine is used in an amount of 0.1-1.0 mole, preferably 0.1-0.5 mole, more preferably 0.2-0.25 mole, calculated for 1 mole of formic acid. When using triethylamine, the mole compound [(C₂H₅)₃H]₂·(HCO₂H)₅ corresponding to the molar ratio of 1:2.5 plays an outstanding role.

The reaction may be accomplished without using any solvent, in a mixture containing the secondary or tertiary amine, respectively, preferably triethylamine and formic acid; however, an inert organic solvent such as dimethyl-formamide, acetonitrile, chloroform or ethanol may also be used in this reaction.

The reaction can be carried out at a temperature between 10 and 70 °C, preferably between 20 and 55 °C.

The reduction is selective: only the double bond in position 3 of the 1,3-dioxane-4,6-dione cycle is saturated with hydrogen, no other double bond being present in any position of the compound of general formula (IV) is affected. Thus, e.g. the nitro group, which is otherwise easy to reduce, remains unchanged.

The end of the reaction is usually shown by the dissolution of the compound of general formula (IV) or by cessation of the evolution of carbon dioxide.

The reaction can be carried out in a bottle equipped with a mechanical stirrer. When using a solvent, it is suitable to fit the bottle with a reflux condenser.

The isolation of the reaction product is simple. In most cases, the target product is precipitated by diluting the reaction mixture with water and, after filtration and washing with water, the compound of general formula (I) is obtained with a satisfactory quality. Alternatively, the product may be extracteed from the reaction mixture diluted with water and acidified to pH 1 by using a water-immiscible solvent.

The compounds of general formula (IV) can be prepared in a manner known per se, e.g. according to any variant of the Knoevenagel-Doebner reaction, by using the compounds of general formulae (II) and (III) as starting substances.

On using formic acid and a secondary or tertiary amine as condensing agent, the compound of general formula (IV) is formed in the first step of the consecutive reaction and can be isolated.

The yield of the compound of general formula (I), i.e. the success of the reaction is substantially determined by the structure of the compound of general formula (II) conclusively, by the formation liability of the compound of general formula (IV). The mixture of formic acid and a tertiary amine proved to be a very favourable condensing agent. In the presence of formic acid and a tertiary amine, aldehydes very rapidly react with the compounds of general formula (III): the reaction becomes complete within 10 to 60 minutes. This reaction is unsuccessful with paraformaldehyde or acetaldehyde because of formation of the corresponding Michael adduct. Ketones react more slowly; therefore, it is useful to employ them in an excess related to the compound of general formula (III).

In this reaction, the molar ratio of the compound of general formula (II) to the compound of general formula (III) can be varied from 1:1 to 8:1, suitably from 1.5:1 to 6:1, more suitably from 2:1 to 5:1.

Formic acid is used in an amount of 1 to 25 moles, peferably 1.5 to 18 moles, more preferably 3 to 12 moles, in relation to 1 mol of the compound of general formula (III).

The secondary or tertiary amine is used in relation to formic acid in an amount of 1 to 0.1 mole, preferably 1 to 0.5 mole, more preferably 1 to 0.125 mole.

The reaction proceeds even at room temperature; it is carried out at a temperature betwen 10 and 70 °C, preferably between 20 and 55 °C, more preferably between 45 and 55 °C. The reaction becomes complete at 20-25 °C within 1-13 days, at 45-55 °C within 1-13 hours and at 70 °C within 1-4 hours. It is not of purpose to increase the reaction temperature above 70 °C since both the starting substance of general formula (III) as well as the compound of general formula (I) obtained as target product are decomposed at these temperatures.

The reaction can be realized without using any solvent, in a mixture of the secondary or tertiary amine, preferably triethylamine, and formic acid although an inert organic solvent such as dimethylformamide, acetonitrile, chloroform or ethanol may be employed in this reaction, too.

The compounds of general formula (II) are usually produced on industrial scale and are commercially available. The compounds of general formula (III) can be prepared by using the method of Meldrum [J. Chem. Soc. 93, 598 (1908)] or Ott [Liebigs Annalen 401, 159 (1913)].

The invention is illustrated in detail by the following non-limiting Examples. In these Examles the temperature values are given in Celsius degrees. In the data of ¹H-NMR spectra the numeric values mean chemical shifts in ppm units.

### Example 1

### Preparation of 5-(3,4-dimethoxyphenylmethyl)-2,2-dimethyl-1,3-dioxane-4,6-dione

24.3 g (33.6 ml, 0.24 mol) of triethylamine were drop-wise added to 27.6 g (22.6 ml, 0.6 mol) of formic acid under stirring and cooling to give a mixture of 50 ml volume.

7.2 g (0.05 mol) of 2,2-dimethyl-1,3-dioxane-4,6-dione (Meldrum's acid) and 8.3 g (0.05 mol) of veratraldehyde were weighed into a flask equipped with a stirrer and the above mixture of 50 ml volume containing formic acid and triethylamine was added. The reaction mixture was stirred at room temperature for 30 minutes, then heated to 45-50 °C during 30 minutes and stirred at the same temperature for 12 hours. After cooling the reaction mixture to room temperature, 25 ml of water were dropped in while stirring, then the mixture was left to stand at 5-10 °C for 16 hours. The mixture was filtered, the precipitate was suspended twice in 12 ml of ethanol each on the filter, washed with water 3 times and dried at 35-40 °C under reduced pressure.

A yield of 11 g (74.8 %) was obtained, m.p.: 141-143 °C.

After combining the mother liquor with the washings, 350 ml of water were added, the pH value was adjusted to 1-2 with concentrated hydrochloric acid and the mixture was allowed to stand for 16 hours. After filtering, the precipitate was washed with water.

A yield of 0.5 g (3.4 %) was obtained, m.p.: 138-141 °C.

Thus, the overall yield amounted to 78.2 %.

After recrystallizing a small sample of the product from a 1:1 mixture of chloroform and methanol, the melting point raised to 142-144 °C.
IR (KBr): 1785, 1750 cm⁻¹ (CO).
¹H-NMR (DMSO, δ ppm): 1.58 (s, 3H, CH₃), 1.80 (s, 3H, CH₃), 3.24 (d, 2H, CH₂), 3.70 (s, 6H, 2 OCH₃), 4.73 (t, 1H, CH), 6.73-6.88 (m, 3H, aromatic).

| Analysis: | | |
|---|---|---|
| Calculated: | C 61.2; | H 6.16 %; |
| Found: | C 60.9; | H 6.23 %. |

### Example 2

### Preparation of 5-(3,4-dimethoxyphenylmethyl)-2,2-dimethyl-1,3-dioxane-4,6-dione

After adding 43.2 g (0.3 mol) of Meldrum's acid, 49.85 g (0.3 mol) of veratraldehyde and 25 ml of the mixture of formic acid and triethylamine, prepared as described in Example 1, to 200 ml of ethanol, the solution was stirred at 20-25 °C for 40 minutes. The crystalline precipitate was filtered, washed with water and ethanol and dried at 35 °C under reduced pressure to obtain 80.3 g (91.6 %) of 5-(3,4-dimethoxyphenylmethylene)-2,2-dimethyl-1,3-dioxane-4,6-dione, m.p.: 173 °C.

After adding 14.6 g (0.05 mol) of the above product to 50 ml of the mixture of formic acid and triethylamine prepared as described in Example 1, the solution was stirred at 45-50 °C for 12 hours and the product obtained was separated as described in Example 1.

A yield of 12.0 g (81.5 %) was obtained.

The results of identification of the end-product are in agreement with those given in Example 1.

### Example 3

### Preparation of 5-(3,4,5-trimethoxyphenylmethyl)-2,2-dimethyl-1,3-dioxane-4,6-dione

A reaction mixture containing 19.6 g (0.1 mol) of 3,4,5-trimethoxy-benzaldehyde, 14.4 g (0.1 mol) of Meldrum's acid and 100 ml of the mixture of formic acid and triethylamine, prepared as described in Example 1, was stirred in an apparatus equipped with a mechanical stirrer at room temperature for 6 days. After adding 100 ml of water cooled to 0 °C, the pH value of the mixture was adjusted to 1 with concentrated hydrochloric acid. After one hour the product was filtered, washed with water and dried.

A yield of 23.7 g (73.1 %) was achieved, m.p.: 103.5-106 °C.

500 ml of water were added to the mother liquor combined with the washings, then the solution was left to stand at 5-10 °C for 16 hours, then the precipitate was filtered and washed with water.

A yield of 1.6 g (4.9 %) was obtained, m.p.: 104-106 °C.

The overall yield amounted to 78 %.

2 g of the above product were dissolved in 10 ml of acetone at room temperature, stirred with 0.2 g of activated charcoal for 30 minutes, then the charcoal was filtered. Water was dropwise added to the acetone filtrate until a precipitate began to fall out. After standing at 5-10 °C overnight the mixture was filtered and suspended on the filter with a 1:4 by volume mixture of acetone and water to give 1.8 g of pure product, m.p.: 105-106.5 °C.
IR (KBr): 1785, 1745 cm⁻¹ (CO).
¹H-NMR (CDCl₃, δ ppm): 1.51 (s, 3H, CH₃), 1.75 (s, 3H, CH₃), 3.45 (d, 2H, CH₂), 3.75 (t, 1H, CH), 3.81 (s, 3H, OCH₃), 3.85 (s, 6H, 2 OCH₃), 6.56 (s, 2H, aryl).

### Example 4

### Preparation of 5-(4-methylphenylmethyl)-2,2-dimethyl-1,3-dioxane-4,6-dione

A reaction mixture containing 12 g (11.8 ml, 0.1 mol) of p-methylbenzaldehyde, 14.4 g (0.1 mol) of Meldrum's acid and 100 ml of the mixture of formic acid and triethylamine, prepared as described in Example 1, was stirred in an apparatus equipped with a stirrer at room temperature for 3 days. The product was isolated according to Example 3 and a small sample thereof was purified as described in Example 3.

A yield of 17.9 g (72.1 %) was obtained, m.p.: 111-112.5 °C.

The quality characteristics of the purified product were as follows:
M.p.: 111.5-112.5 °C.
IR (KBr): 1785, 1750 cm⁻¹ (CO).
¹H-NMR (CDCl₃, δ ppm): 1.51 (s, 3H, CH₃), 1.75 (s, 3H, CH₃), 2.45 (s, 3H, φ-CH₃), 3.46 (d, 2H, CH₂), 3.75 (t, 1H, CH), 7.16 (m, 4H, aromatic).

### Example 5

### Preparation of 5-(3-methoxy-4-hydroxyphenylmethyl)-2,2-dimethyl-1,3-dioxane-4,6-dione

After adding 50 ml of the mixture of formic acid and triethylamine, prepared as described in Example 1, to a mixture of 7.6 g (0.05 mol) of vanillin and 7.2 g (0.05 mol) of Meldrum's acid, the reaction mixture was stirred at room temperature for 7 days. The solution was then stirred with 1 g of activeted charcoal for 30 minutes, then filtered and the charcoal was washed with 50 ml of acetone. After dropping 150 ml of cold water under stirring to the acetone washings combined with the filtrate, the solution was let stand at 5-10 °C for 16 hours, then filtered, washed with water and dried. A yield of 7.1 g (50.7 %) was obtained, m.p.: 111-113 °C.

The mother liquor combined with the washings was extracted twice with 150 ml of chloroform each. After washing the combined chloroform extract with 100 ml of 5 % hydrochloric acid, the chloroform phase was dried over anhydrous sodium sulfate and the chloroform was evaporated under reduced pressure. The oily distillation residue became gradually crystalline at room temperature. After mixing with carbon tetrachloride, it was filtered and washed with carbon tetrachloride.

A yield of 3.85 g (27.5 %) was obtained, m.p.: 103-106 °C.

5 g of the crude product were dissolved in 75 ml of methanol at room temperature. After adding 0.5 g of activated charcoal, it was stirred for 30 minutes and then filtered. After dropwise adding 120 ml of cold water to the filtrate, the mixture was maintained at 5-10 °C for 16 hours, then filtered, suspended in a mixture of methanol and water and again filtered to give 3.8 g of purified product, m.p.: 114-115.5 °C.
IR (KBr): 1795, 1750 cm⁻¹ (CO).
¹H-NMR (DMSO, δ ppm): 1.48 (s, 3H, CH₃), 1.73 (s, 3H, CH₃), 3.44 (d, 2H, CH₂), 3.74 (t, 1H, CH), 3.89 (s, 1H, OH), 6.85 (m, 3H, aromatic).

### Example 6

### Preparation of 5-(2-chlorophenylmethyl)-2,2-dimethyl-1,3-dioxane-4,6-dione

A mixture containing 7 g (5.6 ml, 0.05 mol) of 2-chlorobenzaldehyde), 7.2 g (0.05 mol) of Meldrum's acid and 50 ml of the mixture of formic acid and triethylamine, prepared as described in Example 1, was stirred in a bottle equipped with a mechanical stirrer for 8 hours, maintained at room temperature for 16 hours and then slowly poured into 200 ml of ice-water. After adjusting the pH value to 1 by adding hydrochloric acid of 1:1 dilution the mixture was filtered and the precipitate was washed with water.

A yield of 11.2 g (83.4 %) was achieved, m.p.: 123-127 °C.

A small sample of the above product was recrystallized twice from methanol to give a melting point of 126.5-127.5 °C.
¹H-NMR (DMSO, δ ppm): 1.69 (s, 3H, CH₃), 1.84 (s, 3H, CH₃), 3.88 (d, 2H, CH₂), 4.73 (t, 1H, CH), 7.29 (m, 4H, aromatic).

### Example 7

### Preparation of 5-benzyl-2,2-dimethyl-1,3-dioxane-4,6-dione

100 ml of a mixture of formic acid and triethylamine, prepared as described in Example 1, were added to the mixture of 10.6 g (10.1 ml, 0.1 mol) of benzaldehyde and 14.4 g (0.1 mol) of Meldrum's acid. The reaction mixture spontaneously warmed to 35 °C and the exothermic reaction maintained this temperature for 3.5 hours. The mixture was stirred for additional 3 hours while the reaction mixture cooled to room temperature. The mixture was maintained at this temperature for 16 hours, then poured into 200 ml of ice-water. After adjusting the pH value to 1 with hydrochloric acid of 1:1 dilution, the mixture was left to stand at 5-10 °C for 16 hours, then filtered, washed with water and dried.

A yield of 18.7 g (79.8 %) was obtained, m.p.: 77-80 °C.

After two recrystallisations of a small sample of this product from a mixture of methanol and water, the melting point raised to 80-81 °C.

The melting point as well as the IR and ¹H-NMR spectra of this product proved to be identical with those described in the literature [Synth. Commun. 16, 1701 (1986)].

### Example 8

### Preparation of 5-(2-thienylmethyl)-2,2-dimethyl-1,3-dioxane-4,6-dione

After adding 75 ml of a mixture of formic acid and triethylamine, prepared as described in Example 1, to 11.9 g (0.05 mol) of 5-(2-thienylmethylene)-2,2-dimethyl-1,3-dioxane-4,6-dione, the reaction mixture was stirred at 50-55 °C for 12 hours. After stirring the reaction mixture with 1.2 g of activated charcoal at 50 °C for 15 minutes, the charcoal was filtered out, the filtrate was cooled to room temperature, 80 ml of water were dropwise added and the mixture was let stand at 5-10 °C for 16 hours. Then the precipitate was filtered and washed with water.

A yield of 8.3 g (69.1 %) was achieved, m.p.: 94-96 °C.

To the mother liquor combined with the aqueous washings water was added up to a volume of 500 ml and the mixture was allowed to stand at 5-10 °C for 2 days, then filtered and washed with water.

An additional amount of 1.5 g (12.5 %) of the aimed product were obtained, m.p. 93-96 °C.

The overall yield amounted to 81.6 %.

The product was purified by using an acetone/water mixture as described in Example 3 to give a substance with a melting point of 95-97 °C.

The IR and ¹H-NMR spectra of this product were identical to those described in the literature [Khim. Get. Soed. 1989, 1338; the literature m.p. is 97-98 °C].

### Example 9

### Preparation of 5-cyclohexyl-2,2-dimethyl-1,3-dioxane-4,6-dione

A solution containing 9.8 g (10.5 ml, 0.1 mol) of cyclohexanone, 14.4 g (0.1 mol) of Meldrum's acid and 100 ml of a mixture of formic acid and triethylamine, prepared as described in Example 1, was reacted at room temperature for 2 days, then poured into 200 ml of ice-water. After adjusting the pH value to 1 by adding concentrated hydrochloric acid, the mixture was filtered and the precipitate was washed with water.

A yield of 13.35 g (59.0 %) was obtained, m.p.: 127-129 °C.

A small sample of this product was recrystallized from methanol to give a product melting at 129.5-131.5 °C.

The literature m.p. is 130-131 °C.

The 12 and ¹H-NMR spectra of the product proved to be identical with those described in the literature [Synth. Commun. 16, 1701 (1986)].

### Example 10

### Preparation of 5-isopropyl-2,2-dimethyl-1,3-dioxane-4,6-dione

A solution containing 5.8 g (7.5 ml, 0.1 mol) of acetone, 7.2 g (0.05 mol) of Meldrum's acid and 25 ml of a mixture of formic acid and triethylamine, prepared as described in Example 1, was maintained at room temperature for 6 days. After dropwise adding 40 ml of water, the reaction mixture was let stand at 5-10 °C for 16 hours, then filtered and the precipitate was washed 3 times with water.

A yield of 2.75 g (29.9 %) was obtained, m.p.: 103.5-104 °C.

The IR and ¹H-NMR spectra of the product proved to be identical with those described in the literature [Synth. Commun. 16, 1701 (1986)]; the literature m.p. is 104-105 °C].

### Example 11

### Preparation of 5-isobutyl-2,2-dimethyl-1,3-dioxane-4,6-dione

A solution containing 7.2 g (9.1 ml, 0.1 mol) of isobutyraldehyde, 14.4 g of Meldrum's acid and 100 ml of a mixture of formic acid and triethylamine, prepared as described in Example 1, was stirred at room temperature for 24 hours. The mixture was then poured into 200 ml of ice-water, the pH value was adjusted to 1 by adding concentrated hydrochloric acid and the mixture was left to crystallize at 5 °C for 16 hours. After filtration, the precipitate was washed 3 times with water.

A yield of 10.8 g (53.9 %) was obtained, m.p.: 120-126 °C.

A small sample of the product was dissolved in acetone, clarifified, and water was dropwise added. The precipitate was filtered and recrystallized from boiling methanol, m.p.: 125.5-126.5 °C.

The literature m.p. is 119-120 °C.

The IR and ¹H-NMR spectra of the product proved to be identical with those described in the literature [Synth. Commun. 16, 1701 (1986)].

### Example 12

### Preparation of 5-[2-(1-ethoxycarbonyl)propyl]-2,2--dimethyl-1,3-dioxane-4,6-dione

A solution containing 26 g (25.5 ml, 0.2 mol) of ethyl acetoacetate, 28.8 g (0.2 mol) of Meldrum's acid and 200 ml of a mixture of formic acid and triethylamine, prepared as described in Example 1, was let stand for one week. Subsequently, 700 ml of water were dropwise added, then the precipitate was filtered and washed with water.

A yield of 14.65 g (28.4 %) was achieved, m.p.: 92-93 °C.

After recrystallizing a small sample of the above product from a mixture of acetone and water as described in Example 3, the melting point raised to 94-94.5 °C.
IR (KBr): 1775, 1735 cm⁻¹ (CO).
¹H-NMR (CDCl₃, δ ppm): 1.15 (d, 3H, CH₃), 1.28 (t, 3H, CH₃), 1.78 (s, 3H, CH₃), 1.80 (s, 3H, CH₃), 2.54 (m, 1H, CH), 3.00 (m, 2H, CH₂), 4.15 (m, 3H, CH₂ and CH)

### Example 13

### Preparation of 5-(3,4-dimethoxyphenylmethyl)-2-phenyl-1,3-dioxane-4,6-dione

After stirring 3.4 g (0.01 mol) of 5-(3,4-dimethoxyphenylmethylene)-2-phenyl-1,3-dioxane-4,6-dione (m.p.: 198-200 °C) with 10 ml of a mixture of formic acid and triethylamine, prepared as described in Example 1, at 45-50 °C for 13 hours, the reaction mixture was cooled down, 20 ml of water were added, then the precipitate was filtered and washed with cold water.

A yield of 2.7 g (78.9 %) was achieved, m.p.: 158-161 °C.

A small sample of the product was purified as described in Example 3 to give a product melting at 162-164 °C.
IR (KBr): 1800, 1765 cm⁻¹ (CO)
¹H-NMR (DMSO, δ ppm): 3.20 (d, 2H, CH₂), 3.72 (s, 3H, OCH₃), 3.78 (s, 3H, OCH₃), 4.85 (t, 1H, CH), 6.89-7.55 (m, 9H)

### Example 14

### Preparation of 5-(2-thienylmethyl)-2-phenyl-1,3-dioxane-4,6-dione

After stirring 3.6 g (0.0125 mol) of 5-(2-thienylmethylene)-2-phenyl-1,3-dioxane-4,6-dione (m.p.: 165-166 °C) with 12.5 ml of a mixture of formic acid and triethylamine, prepared as described in Example 1, at 45-50 °C for 10 hours, the reaction mixture was cooled down, 2 ml of water were dropwise added and the mixture was maintained at 5-10 °C for 16 hours, then filtered and washed with water.

A yield of 2.95 g (81.9 %) was obtained, m.p.: 161-164 °C.

After recrystallizing a small sample of the above product according to Example 3, the melting point was raised to 166-167 °C.
IR (KBr): 1800, 1750 cm⁻¹ (CO)
¹H-NMR (DMSO, δ ppm): 3.50 (d, 2H, CH₂), 4.90 (t, 1H, CH), 6.95-7.55 (m, 9H)

### Example 15

### Preparation of 5-(3,4-dimethoxyphenylmethyl)-2,2-pentamethylene-1,3-dioxane-4,6-dione

After stirring 4.15 g (0.025 mol) of veratraldehyde with 4.6 g (0.025 mol) of 2,2-pentamethylene-1,3-dioxane-4,6-dione and 25 ml of a mixture of formic acid and triethylamine, prepared as described in Example 1, at 45-50 °C for 12 hours, the mixture was cooled down, 7 ml of water were dropwise added and the mixture was maintained at 5-10 °C for 16 hours, then the precipitate was filtered and washed with water.

A yield of 7.2 g (86.1 %) was achieved, m.p.: 139-142 °C.

Purification of a small sample of the above product according to Example 3 raised the melting point to 143-144.5 °C.
IR (KBr): 1780, 1745 cm⁻¹ (CO).
¹H-NMR (CDCl₃, δ ppm): 1.60 (m, 10H, 5CH₂), 3.44 (d, 2H, CH₂), 3.75 (t, 1H, CH), 6.84 (m, 3H, aromatic).

### Example 16

### Preparation of 5-cyclohexyl-2,2-pentamethylene-1,3-dioxane-4,6-dione

A solution containing 4.9 g (5.2 ml, 0.05 mol) of cyclohexanone, 4.6 g (0.025 mol) of 2,2-pentamethylene-1,3-dioxane-4,6-dione and 50 ml of a mixture of formic acid and triethylamine, prepared as described in Example 1, was stirred at room temperature for 13 days, then 50 ml of water were added to the reaction mixture, which was then maintained at 5-10 °C for 16 hours, filtered and washed.

A yield of 4.6 g (69.1 %) was achieved, m.p.: 137-139 °C.

Purification of a small sample of the above product according to Example 3 raised the melting point to 138.5-140 °C.
IR (KBr): 1780, 1740 cm⁻¹ (CO).
¹H-NMR (CDCl₃, δ ppm): 1.15-2.41 (m, 21H), 3.35 (d, 1H).

### Example 17

### Preparation of 5-(3,4-dimethoxyphenylmethyl)-2-methyl-2-ethyl-1,3-dioxane-4,6-dione

After stirring 15.8 g (0.1 mol) of 2-methyl-2-ethyl-1,3-dioxane-4,6-dione with 16.6 g (0.1 mol) of veratraldehyde and 100 ml of a mixture of formic acid and triethylamine, prepared as described in Example 1, at 45-50 °C for 12 hours, the reaction mixture was worked up as described in Example 1. The crude product was crystallized from a mixture of acetone and water as described in Example 3.

A yield of 23.1 g (74.9 %) was obtained, m.p.: 110-111 °C.
IR (KBr): 1785, 1745 cm⁻¹ (CO).

### Example 18

### Preparation of 5-(2-butyl)-2,2-dimethyl-1,3-dioxane-4,6-dione

After maintaining a solution containing 36.05 g (44.8 ml, 0.5 mol) of methyl ethyl ketone, 14.4 g (0.1 mol) of Meldrum's acid and 100 ml of a mixture of formic acid and triethylamine, prepared as described in Example 1, at room temperature for 9 days, 300 ml of water were added, the pH value was adjusted to 1 by adding concentrated hydrochloric acid, then the precipitate was filtered and washed with water.

A yield of 3.95 g (19.7 %) was obtained, m.p.: 76-80 °C.

The mother liquor combined with the aqueous washings was extracted 3 times with 130 ml of chloroform each. The combined chloroform extract was dried over anhydrous sodium sulfate and filtered. The chloroform filtrate was evaporated under reduced pressure until it became free of chloroform. After adding 20 ml of ether to the evaporation residue, the mixture was let crystallize at 0 °C for 16 hours, then the crystals were filtered and washed with ether.

The title compound was obtained, m.p.: 78-80.5 °C.

The overall yield amounted to 40.2 %.

Recrystallization of a small sample according to Example 3 raised the melting point to 80.0-80.5 °C. The literature melting point is 79-80 °C [Tetrahedron Lett. 24, 4951 (1983)].
IR (KBr): 1780, 1735 cm⁻¹ (CO).
¹H-NMR (CDCl₃, δ ppm): 0.95 ppm (t, 3H, CH₃), 1.11 ppm (d, 3H, CH₃), 1.64 ppm (m, 2H, CH₂), 1.76 ppm (s, CH₂, 2CH₃), 2.30 ppm (m, 1H, CH), 3.44 ppm (d, 1H, CH)

### Example 19

### Preparation of 5-(3,4-dimethoxyphenylmethyl)-2,2-dimethyl-1,3-dioxane-4,6-dione

The process of Example 1 was followed, except that the reaction was carried out at 70 °C for 4 hours.

The title compound was obtained, m.p.: 137-140 °C.

The IR and ¹H-NMR spectra of the product proved to be identical with that of the compound prepared in Example 1.

### Example 20

### Preparation of 5-(3,4-dimethoxyphenylmethyl)-2,2-dimethyl-1,3-dioxane-4,6-dione

A solution containing 4.15 g (0.025 mol) of vetratraldehyde, 3.6 g (0.025 mol) of Meldrum's acid and 3.1 ml of a mixture of formic acid and triethylamine, prepared as described in Example 1, in 20 ml of chloroform was boiled under reflux for 6 hours. After cooling the reaction mixture to room temperature, 50 ml of water were added and the pH value was adjusted to 1 by adding concentrated hydrochloric acid. The phases were separated and the aqueous layer was extracted with 25 ml of chloroform. The combined chloroform phase was evaporated under reduced pressure and after adding 10 ml of ethanol to the evaporation residue, the mixture was maintained at 5 °C for 16 hours. The crystalline precipitate was filtered and washed with ethanol to give 3.7 g of product which, based on thin-layer chromatography and infrared spectrocopical examinations, was shown to be the mixture of two compounds, namely 5-(3,4-dimethoxyphenylmethyl)-2,2-dimethyl-1,3-dioxane-4,6-dione and 5-(3,4-dimethoxyphenylmethylene)-2,2-dimethyl-1,3-dioxane-4,6-dione. The components of this mixture were separated by fractional crystallization from a mixture of chloroform and methanol.

The product had a
m.p.: 140-143 °C.

The IR spectrum of this product proved to be identical with that of the compound prepared according to Example 1.

### Example 21

### Preparation of 5-(3,4-dimethoxyphenylmethyl)-2,2-dimethyl-1,3-dioxane-4,6-dione

After dropwise adding 9.5 g (9.65 ml, 0.12 mol) of pyridine to 13.8 g (11.3 ml, 0.3 mol) of formic acid under cooling and stirring, 7.3 g (0.025 mol) of 5-(3,4-dimethoxy-phenylmethylene)-2,2-dimethyl-1,3-dioxane-4,6-dione were added to the reaction mixture which was stirred at 45-50 °C for 33 hours, then cooled down to room temperature. After adding 50 ml of ice-water, the pH value was adjusted to 1 by adding concentrated hydrochloric acid. The precipitate was filtered and washed with water to give 6.2 g of product, which proved to be the mixture of the starting substance and the aimed product on the basis of its chromatographical and infrared spectrocopical examination. The aimed product was obtained by fractional crystallization from a mixture of chloroform and methanol.
M.p.: 139-142 °C.

The IR spectrum of this product proved to be identical with that of the compound prepared in Example 1.

### Example 22

### Preparation of 5-di(ethoxycarbonyl)methyl-2,2-dimethyl-1,3-dioxane-4,6-dione

A solution containing 17.4 g (15.5 ml, 0.1 mol) of diethyl mesoxalate, 14.4 g (0.1 mol) of Meldrum's acid and 100 ml of a mixture of formic acid and triethylamine, prepared as described in Example 1, was maintained at room temperature for 13 days. Subsequently, 150 ml of water were added and the mixture was left to stand at 5-10 °C for 16 hours. The precipitate was filtered and washed with water.

A yield of 19.5 g (64.5 %) was obtained, m.p. 114-117 °C.

The mother liquor combined with the washings was adjusted to pH 1 with hydrochloric acid and extracted 3 times with 100 ml of cloroform each. After evaporating the chloroform under reduced pressure, 10 ml of diethyl ether were added to the residue and the mixture was maintained at 0 °C for 16 hours, then filtered and the precipitate was washed with ether.

A yield of 3.75 g (12.4 %) was achieved, m.p.: 112-116 °C.

An overall yield of 23.25 g (76.9 %) was obtained.

Recrystallization of a small sample of the product according to Example 3 raised the melting point to 118.5-119 °C.
IR (KBr): 1740 cm⁻¹ (CO).
¹H-NMR (CDCl₃, δ ppm): 1.30 (t, 6H, 2CH₃), 1.83 (s, 6H, 2CH₃), 4.28 (m, 6H, 2CH₂ and 2CH).

### Example 23

### Preparation of 5-(2- or 4-nitrophenylmethyl)-2,2-dimethyl-1,3-dioxane-4,6-dione

A solution containing 7.56 g (0.05 mol) of 2- or 4-nitrobenzaldehyde, 7.2 g (0.05 mol) of Meldrum's acid and 25 ml of a mixture of formic acid and triethylamine, prepared as described in Example 1, was maintained at 45-50 °C for 12 hours, then cooled to room temperature. After adding 50 ml of water, the mixture was left to stand at 5-10 °C for 16 hours, then filtered and the precipitate was washed with water.

A yield of 11 g (78.8 %) was achieved, m.p.: 139-143 °C.

Recrystallization of a small sample of this product raised the melting point to 144-146 °C.
IR (KBr): 1780, 1730 cm⁻¹ (CO).
¹H-NMR (CDCl₃, δ ppm): 1.66 (s, 3H, CH₃), 1.79 (s, 3H, CH₃), 3.58 (d, 2H, CH₂), 3.84 (t, 1H, CH), 7.58-8.14 (m, 4H, aromatic).

### Example 24

### Preparation of 5-cinnamyl-2,2-dimethyl-1,3-dioxane-4,6-dione

After adding 6.6 g (6.3 ml, 0.05 mol) of cinnamaldehyde and 7.2 g (0.05 mol) of Meldrum's acid to 25 ml of a mixture of formic acid and triethylamine, prepared as described in Example 1, the reaction mixture was maintained at 45-50 °C for 12 hours. After cooling to room temperature, 100 ml of water were added and the pH value was adjusted to 1 by adding concentrated hydrochloric acid. The mixture was maintained at 5-10 °C for 16 hours, then the liquid phase was decanted from the product precipitated in plastic form. Subsequently, the plastic substance was dissolved in acetone at room temperature and, after adding 1 g of activated charcoal, it was stirred for 1 hour, then the charcoal was filtered. Water was dropwise added to the filtrate until permanent opalization, then the mixture was left to stand at 5-10 °C for 7 days. The crystalline precipitate was filtered and washed with water.

A yield of 8.55 g (65.7 %) was obtained, m.p.: 103-107 °C.

A small sample of this product was recrystallized twice from a mixture of acetone and water according to Examle 3 to give the aimed compound with a melting point of 109-110 °C. The literature melting point is 108-109 °C [Tetrahedron Lett. 25, 2325 (1979)].

The ¹H-NMR spectrum of this product proved to be identical with that known in the literature.

### Example 25

### Preparation of 5-methoxycarbonylmethyl-2,2-dimethyl-1,3-dioxane-4,6-dione

A solution containing 8.8 g (0.1 mol) of methyl glyoxalate, 14.4 g (0.1 mol) of Meldrum's acid and 100 ml of a mixture of formic acid and triethyl-amine, prepared as described in Example 1, was maintained at room temperature for 13 days, then poured into 300 ml of ice-water. The pH value was adjusted to 2 by adding concentrated hydrochloric acid, then the mixture was extracted twice with 200 ml of chloroform each. After drying the combined chloroform phase over anhydrous sodium sulfate and filtering the drying agent, the filtrate was evaporated under reduced pressure until it became chloroform-free. After adding 25 ml of diethyl ether to the honeylike evaporation residue, the mixture was left to stand at 5-10 °C for 3 days. The crystalline precipitate was filtered and washed with ether, then with ethyl acetate.
M.P.: 106-109 °C.

Recrystallization of a small sample of the product from acetone raised the melting point to 111-112 °C.
IR (KBr): 1780, 1740 cm⁻¹ (CO).
¹H-NMR (CDCl₃, δ ppm): 1.83 (s, 6H, 2CH₃), 3.18 (d, 2H, CH₂), 3.74 (s, 3H, CH₃), 3.83 (t, 1H, CH).

### Example 26

### Preparation of 5-(3,4-dimethoxyphenylmethyl)-2,2-dimethyl-1,3-dioxane-4,6-dione

After stirring 4.15 g (3.025 mol) of veratraldehyde with 3.6 g (0.025 mol) of Meldrum's acid, 25 ml of dimethylformamide and 6.25 ml of a mixture of formic acid and triethylamine, prepared as described in Example 1, at 45-50 °C for 12 hours, the reaction mixture was cooled to room temperature, 75 ml of water cooled to 0 °C were added and the pH value was adjusted to 1 by adding concentrated hydrochloric acid. After maintaining at 5-10 °C for 16 hours, the precipitate was filtered and washed with water.

A yield of 5.85 g (79.5 %) was obtained, m.p.: 139-142 °C.

The IR spectrum of this product proved to be identical with that of the compound prepared in Example 1.

### Example 27

### Preparation of 5-(3,4-dimethoxyphenylmethyl)-2,2-dimethyl-1,3-dioxane-4,6-dione

A reaction mixture containing 7.3 g (0.025 mol) of 5-(3,4-dimethoxyphenylmethylene)-2,2-dimethyl-1,3-dioxane-4,6 -dione, 20 ml of acetonitrile and 2.1 ml of a mixture of formic acid and triethylamine, prepared as described in Example 1, was maintained at 45-50 °C for 12 hours. Subsequently the acetonitrile was distilled off under reduced pressure. After adding 50 ml of ice-water to the residue, the pH value was adjusted to 1 by adding concentrated hydrochloric acid. The mixture was maintained at 5-10 °C for 16 hours, then filtered and the precipitate was washed with water.

A yield of 6.86 g (93.2 %) was obtained, m.p.: 137-141 °C.

Based on thin-layer chromatography and infrared spectroscopical examination, the product contained about 10 % of starting substance as contamination.

### Example 28

### Preparation of 5-(3,4-dimethoxyphenylmethyl)-2,2-dimethyl-1,3-dioxane-4,6-dione

A reaction mixture containing 4.15 g (0.025 mol) of veratraldehyde, 3.6 g (0.025 mol) of Meldrum's acid, 20 ml of dimethylformamide, 1.73 g (1.4 ml, 0.0375 mol) of formic acid and 0.13 g (0.18 ml, 0.00125 mol) of triethylamine was heated at 45-50 °C for 12 hours, then cooled to room temperature and filtered. Thus, 2.5 g of a product with a melting point of 172-174 °C were obtained which proved to be 5-(3,4-dimethoxyphenylmethylene)-2,2-dimethyl-1,3-dioxane-4,6-dione on the basis of thin-layer chromatography and infrared spectroscopical investigation. After adding 50 ml of water to the filtrate, maintaining it at 5-10 °C for 16 hours and filtering, 2.6 g of a product were obtained which was found to be a mixture of 5-(3,4-dimethoxyphenylmethyl)-2,2-dimethyl-1,3-dioxane-4,6-dione and 5-(3,4-dimethoxyphenylmethylene)-2,2-dimethyl-1,3-dioxane-4,6-dione. The aimed product was obtained by fractional crystallization from a mixture of chloroform and methanol.
M.p.: 140-143 °C.

The last product proved to be identical with the compound prepared according to Example 1 on the basis of thin-layer chromatography and infrared spectroscopical examination.

### Example 29

### Preparation of 5-(3,4-dimethoxyphenylmethyl)-2,2-dimethyl-1,3-dioxane-4,6-dione

4.15 g (0.025 mol) of veratraldehyde were reacted with 3.6 g (0.025 mol) of Meldrum's acid, 20 ml of ethanol and 6.25 ml of a mixture of formic acid and triethylamine, prepared as described in Example 1, in such a way that the temperature of the reaction mixture was increased to 45-50 °C during 30 minutes and the mixture was stirred at the same temperature for 25 hours. After cooling to room temperature and maintaining at this temperature for 16 hours, the reaction mixture was filtered, then the precipitate was washed with ethanol on the filter and dried. The product filtered out weighed 1.3 g and melted at 170-171.5 °C. Based on its infrared spectrum, this product proved to be 5-(3,4-dimethoxyphenylmethylene)-2,2-dimethyl-1,3-dioxane-4,6-dione.

The ethanol content of the mother liquor combined with the ethanolic washings was removed by evaporation under reduced pressure. 50 ml of water were added to the evaporation residue, the pH value was adjusted to 1 by adding hydrochloric acid of 1:1 dilution and the mixture obtained was maintained at 5 °C for 16 hours. The crystalline precipitate was filtered and washed on the filter with water. The wet product weighing 5.3 g was dissolved in 60 ml of acetone at room temerature then stirred with 0.3 g of activated charcoal at room temperature for 30 minutes. After removing the charcoal by filtration, the filtrate was cooled to 5 °C, 120 ml of water were added dropwise under cooling and then the solution was left to stand at 5 °C for 16 hours. After filtering, the crystalline precipitate was washed with an acetone/water mixture of 1:2 volume ratio and dried.

A yield of 2.65 g (36 %) was obtained, m.p.: 140-143 °C.

The infrared spectrum of the product proved to be identical with that of the compound prepared according to Example 1.

### Example 30

### Preparation of 5-(4-methylthiophenylmethyl)-2,2-dimethyl-1,3-dioxane-4,6-dione

After adding 7.2 g (0.05 mol) of Meldrum's acid and 7.6 g (6.5 ml, 0.05 mol) of 4-methylthiobenzaldehyde to 25 ml of a mixture of formic acid and triethylamine, prepared as described in Example 1, the reaction mixture was maintained at room temperature for 7 days, then 50 ml of water were added and the mixture was left to stand at 5 °C for 24 hours. The precipitate was filtered and washed with water. The wet product was dissolved in 150 ml of acetone at room temperature and stirred with 1 g of activated charcoal for 30 minutes. After removing the charcoal by filtration, 100 ml of water were dropped to the filtrate. The mixture was maintained at 5 °C for 24 hours, then filtered and washed with a mixture of acetone and water. The precipitate obtained by adding additional water to the mother liquor was filtered and repeatedly purified as described above.

A yield of 10.4 g (74.2 %) was obtained, m.p.: 91-92 °C.
IR (KBr): 1790, 1745 cm⁻¹ (CO).
¹H-NMR (CDCl₃, δ ppm): 1.54 (s, 3H, CH₃), 1.73 (s, 3H, CH₃), 2.44 (s, 3H, SCH₃), 3.44 (d, 2H, CH₂), 3.74 (t, 1H, CH), 7.20 (m, 4H, aromatic).

### Example 31

### Preparation of 5-(4-dimethylaminophenylmethyl)-2,2-dimethyl-1,3-dioxane-4,6-dione

After adding 25 ml of dimethylformamide and 6.9 g (0.025 mol) of 5-(4-dimethylaminophenylmethylene)-2,2-dimethyl-1,3-dioxane-4,6-dione to 25 ml of a mixture of formic acid and triethylamine, prepared as described in Example 1, the reaction mixture was stirred at room temperature for 1 week, then 100 ml of water were added and the mixture was extracted 3 times with 100 ml of chloroform each. The combined chloroform phase was washed with 50 ml of saturated saline solution. The organic phase was dried over anhydrous sodium sulfate and after filtering out the drying agent, the solution was concentrated to 20 ml under reduced pressure. After adding 60 ml of petroleum ether to the residue the mixture was left to stand at -10 °C for 7 days. After filtration the crystals were washed with petroleum ether, dried and recrystallized from a mixture of acetone and ethanol.

A yield of 2.15 g (31.0 %) was obtained, m.p.: 108.5--109.5 °C.
IR (KBr): 1795, 1755 cm⁻¹ (CO).
¹H-NMR (δ ppm): 1.45 (s, 3H, CH₃), 1.71 (s, 3H, CH₃), 2.91 (s, 6H, N(CH₃)₂), 3.41 (d, 2H, CH₂), 3.71 (t, 1H, CH), 6.66 and 7.19 (m, 4H, aromatic).

### Example 32

### Preparation of 5-cyclopentyl-2,2-dimethyl-1,3-dioxane-4,6-dione

A reaction mixture containing 14.4 g (0.1 mol) of Meldrum's acid, 8.4 g (8.9 ml, 0.10 mol) of cyclopentanone, 50 ml of a mixture of formic acid and triethylamine, prepared as described in Example 1, and 0.2 ml of piperidine was maintained at room temperature for 7 days, then 100 ml of water were added and the mixture was maintained at 5 °C for 1 day. The crystalline precipitate was filtered, washed with water and dried at room temperature under reduced pressure.

Thus, 12.2 g of product were obtained, which proved to be the mixture of 5-cyclopentylidene- and 5-cyclopentyl-2,2-dimethyl-1,3-dioxane-4,6-dione on the basis of thin-layer chromatography and infrared spectroscopical investigation. After dissolving this crude product in acetone, the two components were separated by fractional precipitation with water.
M.p.: 99-100
°C. The literature m.p. is 92-93 °C [Tetrahedron Lett. 24, 4951 (1983)].
IR (KBr): 1780, 1760 cm⁻¹ (CO).
¹H-NMR (CDCl₃, δ ppm): 1.76 (m, 14H), 2.70 (m, 1H), 3.60 (d, 1H).

### Example 33

### Preparation of 5-(3,4-dimethoxyphenylmethyl)-2,2-dimethyl-1,3-dioxane-4,6-dione

After dropwise adding 27.2 g (33.1 ml, 0.24 mol) of N-ethylpiperidine to 27.6 g (22.6 ml, 0.6 mol) of formic acid under stirring and cooling, 7.2 g (0.05 mol) of Meldrum's acid and 8.3 g (0.05 mol) of veratraldehyde were added to the above mixture, then the procedure described in Example 1 was followed.

A yield of 11.55 g (78.5 %) was obtained, m.p.: 141-143 °C.

The infrared spectrum of this product proved to be identical with that of the compound prepared according to Example 1.

### Example 34

### Preparation of 5-(3,4-dimethoxyphenylmethyl)-2,2-dimethyl-1,3-dioxane-4,6-dione

After dropwise adding 2.55 g (3 ml, 0.03 mol) of piperidine to a mixture of 5.75 g (4.7 ml, 0.125 mol) of formic acid and 25 ml of dimethylformamide, 4.15 g (0.025 mol) of veratraldehyde and 3.6 g (0.025 mol) of Meldrum's acid were added. The reaction mixture was stirred at room temperature for 30 minutes, then heated to 45 °C during 30 minutes and stirred at 45-50 °C for 12 hours. After cooling the reaction mixture to room temperature, 75 ml of water of 0 °C temperature were added and the pH value was adjusted to 1 by adding hydrochloric acid of 1:1 dilution. The mixture was maintained at 5 °C for 24 hours, then the crystalline precipitate was filtered and washed with water.

A yield of 4.70 g (63.9 %) was obtained, m.p.: 139-141 °C.

Based on its infrared spectrum, this product proved to be identical with the compound prepared according to Example 1.

### Example 35

### Preparation of 5-(3,4-dimethoxyphenylmethyl)-2,2-dimethyl-1,3-dioxane-4,6-dione

After dropwise adding 17.55 g (25 ml, 0.24 mol) of diethylamine to 27.6 g (22 ml, 0.6 mol) of formic acid while stirring and cooling, 7.2 g (0.05 mol) of Meldrum's acid and 8.3 g (0.05 mol) of veratraldehyde were added to the above mixture. The reaction mixture was stirred at room temperature for 30 minutes, then warmed to 45-50 °C during 30 minutes and maintained at 45-50 °C for 14 hours. The reaction mixture was cooled to room temperature and maintained at the same temperature for 1 day. The crystalline precipitate was filtered, washed with ethanol and dried. Thus, 9.95 g of product were obtained, a third part of which was the title compound and about two thirds were 5-(3,4-dimethoxyphenylmethylene)-2,2-dimethyl-1,3-dioxane-4,6-dione. This mixed product was subjected to fractional crystallization from a mixture of chloroform and methanol to give 3 g of title compound. The original mother liquor and the ethanolic washings were combined, supplemented with water up to 500 ml and after adjusting the pH value to 1 by adding concentrated hydrochloric acid, the mixture was maintained at 5 °C for 24 hours. The crystalline precipitate was filtered and washed with water. The filter-wet product was dissolved in acetone, stirred with 0.2 g of activated charcoal at room temperature, then the charcoal was removed by filtration. After adding water to the filtrate, the crystalline precipitate was filtered and washed with a mixture of acetone and water to give 1.75 g of the aimed compound.

Thus, a yield of 4.75 g (32.3 %) was obtained, m.p.: 140-143 °C.

The infrared spectrum of this product proved to be identical with that of the compound prepared according to Example 1.

### Example 36

### Preparation of 5-(3,4-dimethoxyphenylmethyl)-2,2-dimethyl-1,3-dioxane-4,6-dione

After dropwise adding 20.9 g (20.9 ml, 0.24 mol) of morpholine to a mixture of 27.6 g (22.6 ml, 0.6 mol) of formic acid and 50 ml of dimethylformamide under stirring and cooling, 7.2 g (0.05 mol) of Meldrum's acid and 8.3 g (0.05 mol) of veratraldehyde were added. The reaction mixture was stirred at room temperature for 30 minutes, then the temperature was raised to 45 °C during 30 minutes. The mixture was stirred at 45-50 °C for 12 hours, then after cooling to room temperature 100 ml of water were added and the pH value was adjusted to 1 by adding concentrated hydrochloric acid. After maintaining the mixture at 5 °C for 24 hours, the crystalline precipitate was filtered and washed with water.

A yield of 6.35 g (43.1 %) was achieved, m.p.: 139-142 °C.

The infrared spectrum of this product proved to be identical with that of the compound prepared according to Example 1.

## Claims

1. A process for the preparation of compounds of the general formula (I), 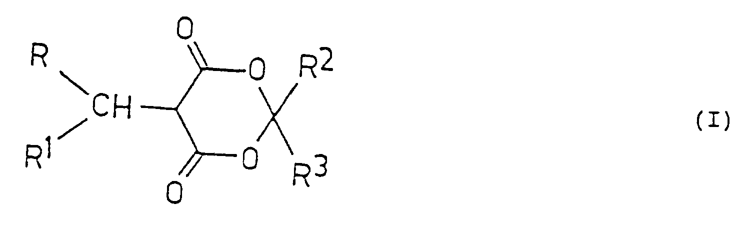 wherein
R means hydrogen, C₁₋₄alkyl or (C₁₋₅alkoxy)carbonyl group;
R¹ stands for a C₁₋₆ alkyl, (C₁₋₅alkyl)carbonyl, (C₁₋₅alkoxy)carbonyl or (C₁₋₅alkoxy)carbonyl-(C₁₋₄alkyl) group; or a cyclohexyl group; or a cyclopentyl group; or a phenyl, furyl or thienyl group, optionally mono-, di- or trisubstituted by halogen, C₁₋₄alkoxy, C₁₋₄alkyl, nitro, C₁₋₄alkylthio, di(C₁₋₄alkyl)amino or hydroxy groups; or a b-CH=CH group, wherein b is a phenyl group; or
R and R¹ together form a straight-chain C₄₋₅alkylene group;
R² represents a C₁₋₅alkyl or phenyl group;
R³ means hydrogen or a C₁₋₄alkyl group; or
R² and R³ together form a pentylene group,
by reacting a compound of general formula (II) 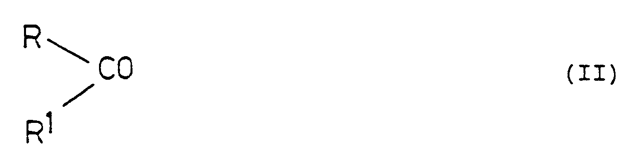 with a compound of general formula (III) 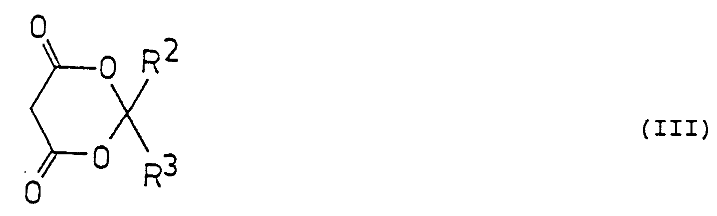 and/or reducing a compound of the general formula (IV), 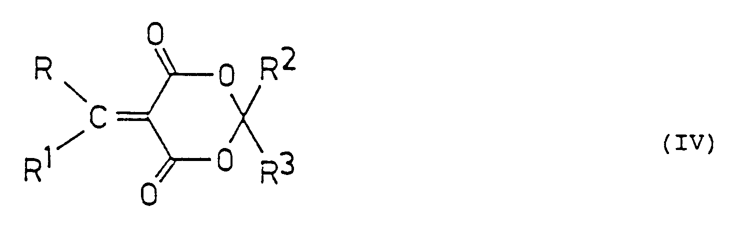 wherein R, R¹, R² and R³ are as defined above, at a temperature of 10 to 70°C, characterized by working in the presence of formic acid and [a] secondary and/or tertiary amine(s).

2. A process as claimed in claim 1, which **comprises** using [an] amine(s) di- or trisubstituted by C₁₋₄alkyl group(s) or [a] cyclic amine(s) optionally N-substituted by a C₁₋₄alkyl group as secondary and/or tertiary mine(s).

3. A process as claimed in claim 1, which **comprises** using diethylamine, triethylamine, pyridine, piperidine, N-ethylpiperidine and/or morpholine as secondary and/or tertiary amine(s).

4. A process as claimed in claim 1, which **comprises** using formic acid in an amount of 1-20 moles, more preferably 3-12 moles, most preferably 6-12 moles in relation to 1 mole of the compound of general formula (IV).

5. A process as claimed in claim 1, which **comprises** using the secondary or tertiary amine in an amount of 0.1-1.0, preferably 0.1-0.5, more preferably 0.2-0.25 mole in relation to 1 mole of formic acid.

6. A process as claimed in claim 1, which **comprises** carrying out the reduction without using any solvent.

7. A process as claimed in claim 1, which **comprises** carrying out the reduction in the presence of an inert organic solvent, suitably dimethylformamide, acetonitrile, cloroform or ethanol.

8. A process as claimed in claim 1, which **comprises** carrying out the reduction at a temperature of 10-70 °C, preferably 20-55 °C.

9. A process as claimed in claim 1, in which the compound of formula (IV) is prepared by reacting a compound of formula (II) with a compound of formula (III) in the presence of formic acid and a secondary or tertiary amine.

10. A process as claimed in claim 1, in which the synthesis is carried out in a "one-pot" reaction, without isolating the intermediate product of formula (IV).

## Patentansprüche

1. Verfahren zur Herstellung von Verbindungen der allgemeinen Formel (I) 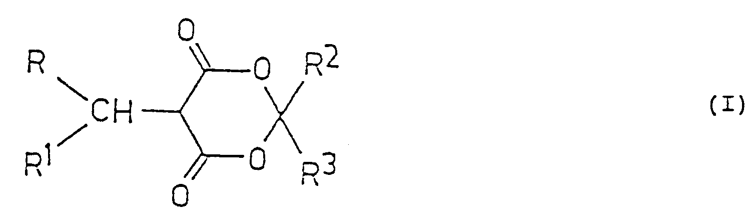 worin
R Wasserstoff, eine C₁₋₄-Alkyl- oder eine (C₁₋₅-Alkoxy)carbonylgruppe bedeutet;
R¹ für eine C₁₋₆-Alkyl-, (C₁₋₅-Alkyl)carbonyl-, (C₁₋₅-Alkoxy)carbonyl- oder (C₁₋₅-Alkoxy)carbonyl-(C₁₋₄-alkyl)gruppe; oder für eine Cyclohexylgruppe; oder für eine Cyclopentylgruppe; oder für eine Phenyl-, Furyl- oder Thienylgruppe, gegebenenfalls mit Halogen, C₁₋₄-Alkoxy-, C₁₋₄-Alkyl-, Nitro-, C₁₋₄-Alkylthio-, Di(C₁₋₄-Alkyl)amino- oder Hydroxygruppen mono-, di- oder trisubstituiert; oder für eine b-CH=CH-Gruppe, worin b eine Phenylgruppe ist, steht; oder
R und R¹ zusammen eine geradkettige C₄₋₅-Alkylengruppe bilden;
R² eine C₁₋₅-Alkyl- oder Phenylgruppe repräsentiert;
R³ Wasserstoff oder eine C₁₋₄-Alkylgruppe bedeutet; oder
R² und R³ zusammen eine Pentylengruppe bilden,
indem eine Verbindung der allgemeinen Formel (II) 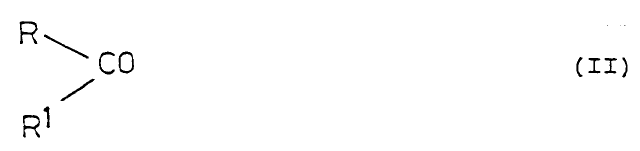 mit einer Verbindung der allgemeinen Formel (III) umgesetzt wird 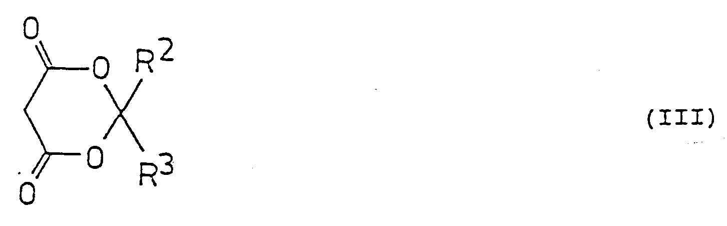 und/oder eine Verbindung der allgemeinen Formel (IV) reduziert wird 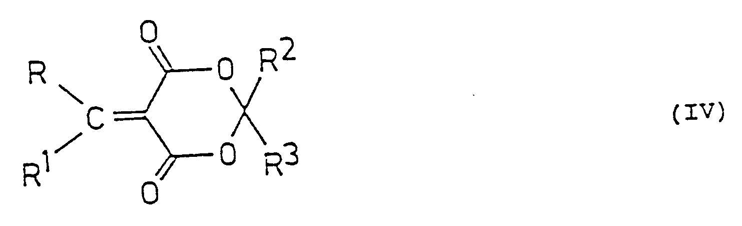 worin R, R¹, R² und R³ die oben definierte Bedeutung haben, bei einer Temperatur von 10 - 70°C, **dadurch gekennzeichnet**, daß in Gegenwart von Ameisensäure und (einem) sekundären und/oder tertiären Amin(en) gearbeitet wird.

2. Verfahren nach Anspruch 1, welches die Verwendung eines oder mehrerer durch (eine) C₁₋₄-Alkylgruppe(n) di- oder trisubstituierter Amine oder eines oder mehrerer cyclischer Amine, gegebenenfalls durch eine C₁₋₄-Alkylgruppe N-substituiert, als sekundäre und/oder tertiäre Amine umfaßt.

3. Verfahren nach Anspruch 1, welches die Verwendung von Diethylamin, Triethylamin, Pyridin, Pideridin, N-Ethylpiperidin und/oder Morpholin als sekundäre(s) und/oder tertiäre(s) Amin(e) umfaßt.

4. Verfahren nach Anspruch 1, welches die Verwendung von Ameisensäure in einer Menge von 1 bis 20 mol, weiter bevorzugt 3 bis 12 mol, am stärksten bevorzugt 6 - 12 mol, bezogen auf 1 mol der Verbindung der allgemeinen Formel (IV) umfaßt.

5. Verfahren nach Anspruch 1, welches die Verwendung des sekundären oder tertiären Amins in einer Menge von 0,1 bis 1,0, vorzugsweise 0,1 bis 0,5, weiter bevorzugt 0,2 bis 0,25 mol, bezogen auf 1 mol Ameisensäure umfaßt.

6. Verfahren nach Anspruch 1, welches die Durchführung der Reduktion ohne Verwendung irgendeines Lösungsmittels umfaßt.

7. Verfahren nach Anspruch 1, welches die Durchführung der Reduktion in Gegenwart eines inerten organischen Lösungsmittels, geeigneterweise Dimethylformamid, Acetonitril, Chloroform oder Ethanol, umfäßt.

8. Verfahren nach Anspruch 1, welches die Durchführung der Reduktion bei einer Temperatur von 10 - 70°C, vorzugsweise 20 - 55°C, umfaßt.

9. Verfahren nach Anspruch 1, bei welchem die Verbindung der Formel (IV) hergestellt wird, indem eine Verbindung der Formel (II) mit einer Verbindung der Formel (III) in Gegenwart von Ameisensäure und einem sekundären oder tertiären Amin umgesetzt wird.

10. Verfahren nach Anspruch 1, bei dem die Synthese in einer 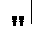 Ein-Topf"-Reaktion durchgeführt wird, ohne Isolieren des Zwischenproduktes der Formel (IV).

## Revendications

1. Procédé pour la préparation de composés de formule générale (I) : 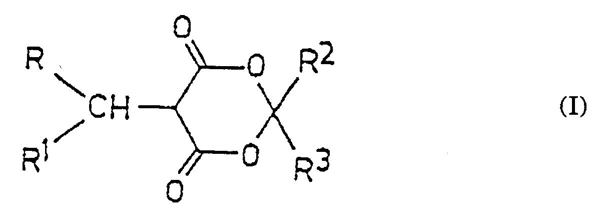 dans laquelle :
R est un atome d'hydrogène, un groupe alkyle en C₁₋₄ ou un groupe alcoxy (en C₁₋₅)-carbonyle;
R¹ est un groupe alkyle en C₁₋₆, un groupe alkyl-(en C₁₋₅)-carbonyle, un groupe alcoxy-(en C₁₋₅)-carbonyle ou un groupe alcoxy-(en C₁₋₅)-carbonyl-alkyl-(en C₁₋₄); ou un groupe cyclohexyle; ou un groupe cyclopentyle; ou un groupe phényle, furyle ou thiényle, éventuellement mono-, di- ou tri-substitué par des atomes d'halogène, des groupes alcoxy en C₁₋₄, alkyle en C₁₋₄, nitro, alkylthio en C₁₋₄, dialkylamino en C₁₋₄ ou hydroxy; ou un groupe b-CH=CH, dans lequel b représente un groupe phényle; ou
R et R¹ forment ensemble un groupe alkylène en C₄₋₅ à chaîne droite;
R² représente un groupe alkyle en C₁₋₅ ou phényle;
R³ représente un atome d'hydrogène ou un groupe alkyle en C₁₋₄; ou
R² et R³ forment ensemble un groupe pentylène;
par réaction d'un composé de formule générale (II): 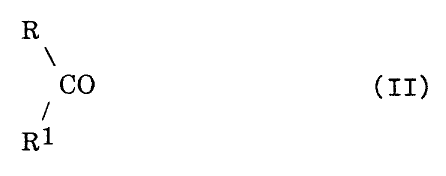 avec un composé de formule générale (III) : 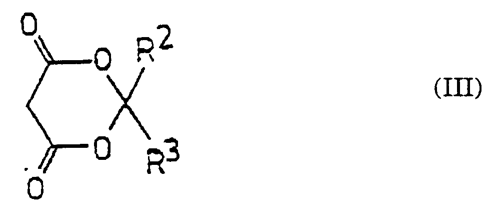 et/ou réduction d'un composé de formule générale (IV) : 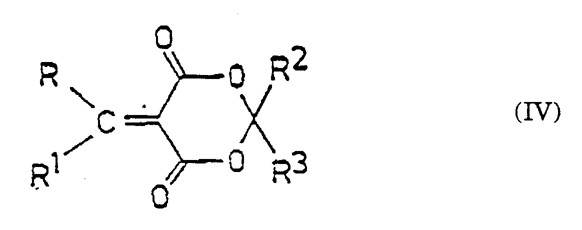 dans laquelle R, R¹ R² et R³ sont tels que définis plus haut, à une température de 10 à 70°C, caractérisé en ce que le procédé est conduit en présence d'acide formique et d'amine secondaire et/ou d'amine tertiaire.

2. Procédé suivant la revendication 1, qui comprend l'utilisation d'une ou plusieurs amines di- ou trisubstituées par un ou des groupes alkyles en C₁₋₄, ou d'une ou plusieurs amines cycliques éventuellement N-substituées par un groupe alkyle en C₁₋₄ en tant qu'amine secondaire et/ou amine tertiaire.

3. Procédé suivant la revendication 1, qui comprend l'utilisation de diéthylamine, de triéthylamine, de pyridine, de pipéridine, de N-éthylpipéridine et/ou de morpholine en tant qu'amine secondaire et/ou amine tertiaire.

4. Procédé suivant la revendication 1, qui comprend l'utilisation d'acide formique en une quantité de 1 à 20 moles, de préférence de 3 à 12 moles et plus avantageusement de 6 à 12 moles pour 1 mole du composé de formule générale (IV).

5. Procédé suivant la revendication 1, qui comprend l'utilisation de l'amine secondaire ou tertiaire en une quantité de 0,1 à 1,0 mole, de préférence de 0,1 à 0,5 mole et plus avantageusement de 0,2 à 0,25 mole pour 1 mole d'acide formique.

6. Procédé suivant la revendication 1, qui comprend la réalisation de la réduction sans utilisation de solvant.

7. Procédé suivant la revendication 1, qui comprend la réalisation de la réduction en présence d'un solvant organique inerte, convenablement du diméthylformamide, de l'acétonitrile, du chloroforme ou de l'éthanol.

8. Procédé suivant la revendication 1, qui comprend la réalisation de la réduction à une température de 10 à 70°C, de préférence de 20 à 55°C.

9. Procédé suivant la revendication 1, dans lequel le composé de formule (IV) est préparé par réaction d'un composé de formule (II) avec un composé de formule (III) en présence d'acide formique et d'une amine secondaire ou tertiaire.

10. Procédé suivant la revendication 1, dans lequel la synthèse est conduite au cours d'une réaction en "un pot", sans isolation du produit intermédiaire de formule (IV).
